# EUROPÄISCHE PATENTSCHRIFT

(11) **EP 0 794 736 B1**
(45) Veröffentlichungstag und Bekanntmachung des Hinweises auf die Patenterteilung: **24.03.1999**
(21) Anmeldenummer: 95940162.1
(22) Anmeldetag: 30.11.1995
(51) Int. Cl.: A61B 17/68, F16B 13/12

(54) **OSTEOSYNTHETISCHES BEFESTIGUNGSELEMENT**
OSTEOSYNTHETIC SECURING ELEMENT
ELEMENT DE FIXATION OSTEOSYNTHETIQUE

(30) Priorität: 30.11.1994 DE 4444510
(43) Veröffentlichungstag der Anmeldung: 17.09.1997
(73) Patentinhaber: Lob, Günter, 81377 München (DE)
(72) Erfinder: Lob, Günter, 81377 München (DE)
(74) Vertreter: Christiansen, Henning, Dipl.-Ing.
(86) Internationale Anmeldenummer: DE9501768
(87) Internationale Veröffentlichungsnummer: WO9616607

(56) Entgegenhaltungen:
- EP-A- 0 409 364
- DE-U- 8 505 817
- DE-U- 9 113 591
- DE-U- 9 210 543
- US-A- 1 752 752

## Beschreibung

Die Erfindung betrifft ein Befestigungselement der im Oberbegriff des Anspruchs 1 angegebenen Art.

Aus der EP-B-409 364 ist ein Verbindungselement für die Osteosynthese bekannt, welches als aufspreizbare, elastisch verformbare Hülse ausgebildet ist. Die Hülse weist einen von einer Stirnfläche aus abnehmenden Durchmesser auf und ist mit einem sich in Längsrichtung erstreckenden Schlitz versehen. Das Aufspreizen der Hülse erfolgt durch einen rotationssymmetrisch ausgebildeten, sich in Eintreibrichtung vorzugsweise konisch verjüngenden Stift, dessen Außendurchmesser größer als der kleinste und kleiner als der größte Außendurchmesser der Hülse ist. An der Peripherie der Hülse sind verschiedenartige Verzahnungselemente vorgesehen, welche beispielsweise aus sägezahnförmigen Widerhaken, wendelförmigen oder konzentrischen Vorsprüngen bestehen.

Bei Eintreiben des Stiftes wird die äußere Wandung der in eine entsprechende Bohrung in den zu verbindenden Knochenfragmenten eingesetzten Hülse gegen das sie umgebende Knochenmaterial gepreßt und entsprechend verkeilt.

Nachteilig bei dem bekannten Implantat zur osteosynthetischen Verbindung von reponierten Knochenfragmenten ist insbesondere, daß - unabhängig von der Qualität ihrer stabilisierenden Wirkung - vor allem die erreichbare Festigkeit der Verbindung nicht vorhersehbar und in Längsrichtung stark variabel ist.

Außerdem entstehen beim Fixieren dieses Elements Torsionsbeanspruchungen, die in ihren Auswirkungen nicht vorhersehbar sind und die Festigkeit der Verbindung herabsetzen.

Ausgehend von den Mängeln des Standes der Technik, liegt der Erfindung die Aufgabe zugrunde, ein Befestigungselement der eingangs genannten Gattung zu schaffen, mit welchem eine verbesserte osteosynthetische Stabilisierung in Annäherung an die Verankerungseigenschaften eines metallischen Befestigungselements erreichbar ist.

Diese Aufgabe wird mit den kennzeichnenden Merkmalen des Anspruchs 1 gelöst.

Die Erfindung schließt die Erkenntnis ein, daß bei einem in eine Aufnahmeöffnung (Bohrung oder sonstiger Längskanal) einfügbaren ostheosynthetischen Fixationselement zur Erzielung einer festen Verbindung mit dem Knochen eine möglichst gleichmäßige Krafteinleitung erfolgen sollte. Dies betrifft die Fixation von Knochenfragmenten genauso wie die Befestigung von Bändern am Knochen. Entsprechendes gilt auch für die Befestigung von Platten. Um hier die herkömmliche Schraube zu ersetzen, ist ein Element erforderlich, welches allein aufgrund seiner Spreizung eine Krafteinleitung über den gesamten durchquerten Knochebereich ermöglicht, ohne daß es selbst in seiner Festigkeit überbeansprucht wird. Deswegen kommt beispielsweise ein Eindrehen unter Torsionsbeanspruchung wie bei herkömmlichen Metallschrauben nicht in Betracht.

Im Gegensatz zu dem bekannten konischen Spreizelement wird dafür gesorgt, daß die relative Expansion über die gesamte Länge der mit dem Knochenmaterial in Eingriff befindlichen Außenoberfläche um einen möglichst konstanten Betrag erfolgt, der ausreichend ist, um einen sicheren Formschluß der äußeren Profilierung zu erzeugen. Hierbei ist eine zwei-, drei- oder auch mehrfache Teilung in Längsrichtung möglich. Dadurch, daß die Teilelemente in radialer bzw. tangentialer Richtung frei gegeneinander verschieblich sind, werden sie zum Einführen in eine Bohrung in eine komprimierte Position gebracht, bei der die Wandung der Bohrung gerade nicht erreicht wird. Anstelle von Bohrungen mit rundem Querschnitt sind auch andere Längskanäle mit von der Kreisform abweichendem Querschnitt und/oder einer Verjüngung in Einführungsrichtung geeignet.

Mit der Expansion der Teilelemente (angetrieben durch den möglichst über die gesamte Länge der inneren Öffnung anliegende und gleichmäßige ansteigende Keilflächen aufweisenden Spreizkörper) führen diese eine Bewegung in radialer Richtung aus, wobei eine die Wandung über im wesentlichen die gesamte Länge berührende Scheitellinie bei der Spreizbewegung vorangeführt wird und um den vollen Bewegungshub und über die volle Länge in die Wandung eindringt. Bei zwei Teilelementen sind dies zwei gegenüberliegende Außenbereiche, während es bei drei Teilelementen zu einer Struktur kommt, die derjenigen der Backen eines Bohrfutters ähnelt. Hierbei kommt es jedoch im Gegensatz zu diesem nicht auf die Eingriffswirkung in bezug auf einen innenliegenden zylindrischen Bohrer, sondern auf den Eingriff in die äußere Wandung an.

Bei einer bevorzugten Ausführungsform ist damit die äußere Krümmung der Teilelemente so zu wählen, daß sie dem expandierten Querschnitt entspricht. Weil in diesem Fall die Seitenlinien der Teilelemente die Bohrung zuerst tangieren, kann es günstig sein die entsprechenden Bereiche in ihrem Querschnitt geringfügig abnehmen zu lassen, um ein möglichst großes Befestigungselement in eine vorgegebene Bohrung einführen zu können und den relativen Expansionshub möglichst groß werden zu lassen.

Bei polygonaler Teilung ist das Spreizelement entsprechend polygonal auszubilden, wobei für jedes äußere Teilelement eine entsprechende gleichartige Gleitfläche vorzusehen ist.

Bei der Anwendung des erfindungsgemäßen Befestigungselements zu Fixation von Bändern oder dergl. im Bohrloch weist dieses bevorzugt in seinem Umfangsquerschnitt eine Ausnehmung zur klemmenden Einfügung des Bandes o.ä. auf.

Zum Fixieren von Platten und dergleichen dient ein umlaufender flanschartiger Kragen.

Besonders vorteilhaft bei der Erfindung ist der Umstand, daß die Expansion über die gesamte Länge im wesentlichen gleichmäßig erfolgt, so daß keine Torsions- oder Schwenkbewegungen auftreten können, welche eine ungleichmäßige Fixierung mit der Gefahr einer lokalen Überbeanspruchung hervorrufen könnten.

Besonders günstig ist es auch, wenn die Tiefe der äußeren Profilierung dem erreichbaren Hub entspricht, so daß dem verdrängten Knochenmaterial ein Kompressionsbereich innerhalb der Struktur zur Verfügung steht.

Entsprechend einer bevorzugten Ausführungsform weist das Befestigungselement für die Osteosynthese einen aus zwei, einen Hohlraum begrenzenden Teilelementen bestehenden, im wesentlichen stabförmig ausgebildeten Aufnahmekörper und einen Spreizkörper auf. Der zur Verbindung von Knochenfragmenten in einer Knochenbohrung positionierte Aufnahmekörper verändert seine Raumform, wenn der Spreizkörper in den von den Teilelementen umschlossenen, sich über die gesamte Länge des Aufnahmekörpers erstreckenden Hohlraum eingebracht wird. Die Teilelemente des Aufnahmekörper sind nicht fest miteinander verbunden, sondern durch ein Führungselemente derart in einer Position zueinander gehalten, daß bei Einbringen des Spreizkörpers in den Hohlraum eine gleichmäßige Abstandsänderung zwischen dem jeweiligen Teilelement und der Längsachse des Aufnahmekörpers in einer jeweils zu dieser Achse senkrechten Richtung eintritt.

Dadurch wird - wie zuvor erläutert - in vorteilhafter Weise erreicht, daß die Teilelemente durch den Spreizvorgang keiner Verformung unterworfen werden und zwischen der Außenwandung des Befestigungselements und dem Knochenmaterial über die gesamte Länge des Befestigungselements eine im wesentlichen gleichmäßige Flächenpressung erzeugt wird.

Die Führungselemente sind als Gerad-Führung mit einer Zunge und einer die Zunge aufnehmenden Ausnehmung ausgebildet, wobei sich die Führungselemente jeweils an den einander zugewandten Seiten der Teilelemente und in Wirkungs eingriff befinden, wenn das Befestigungselement in eine Knochenbohrung eingesetzt wird.

Der zwischen den Teilelementen des Aufnahmekörpers befindliche Hohlraum weist einen viereckigen, beispielsweise rechteckigen Querschnitt auf, welcher sich entsprechend einer vorteilhaften Weiterbildung der Erfindung einseitig an einem Endbereich in Richtung der Hohlraumöffnung keilartig erweitert. Dadurch wird das Eintreiben eines als stabförmiger Quader ausgebildeten Spreizkörpers während des chirurgischen Eingriffs erheblich erleichert. Der Quaderquerschnitt ist dem rechteckigen Querschnittsprofil des Hohlraums im Aufnahmekörper angepaßt, wobei jedoch das Seiten- oder Höhenmaß im Verhältnis zum Querschnittsprofil des Hohlraums einen größeren Wert aufweist, um die gewünschte Spreizwirkung zu erreichen. Für das Einführen des Spreizkörpers in die sich erweiternde Öffnung des Aufnahmekörpers ist darüberhinaus ein Verjüngen des Spreizkörpers durch die Anordnung einer keilförmigen Anfasung an dessen einzuführendem Ende günstig.

Der viereckige Querschnitt des Hohlraums und des Spreizkörpers sichern im Zusammenwirken mit der Gerad-Führung, daß bei Eintreiben des Spreizkörpers eine Bewegung der Teilelemente des Aufnahmekörpers unter Beibehaltung ihrer Parallelität und ihrer zur Achse des Hohlraums spiegelsymmetrischen Lage erfolgt. Dadurch ist eine gleichmäßige, senkrecht zu dieser Längsachse und zueinander entgegengesetzt gerichtete Bewegung der Teilelemente möglich und bewirkt eine im wesentlichen gleichmäßige Flächenpressung zwischen der Außenwandung des Befestigungselements und dem umgebenden Knochenmaterial über die Gesamtlänge des Befestigungselements.

Das dem keilartig ausgebildeten Endabschnitt gegenüberliegende Ende des Spreizkörpers weist gemäß einer anderen Ausführungsform der Erfindung eine profilierte Wandung auf, welche eine vergrößerte Angriffsfläche für das zeitweilige form- und kraftschlüssige Anschlagen eines chirurgischen Hilfselements bildet, welches für ein bequemes Handhaben des Spreizkörpers vorgesehen ist. Dafür sind sich quer zur Längsachse des Spreizkörpers erstreckende Profilabschnitte mit einem dreieckigen Querschnitt besonders geeignet. Für die Handhabung des Spreizkörpers ist es dabei gleichermaßen wichtig, daß der Aufnahmekörper eine geringere Länge aufweist als der Spreizkörper. Dadurch wird erreicht, daß das profilierte Ende des Spreizkörpers auch dann noch handhabbar ist, wenn dieser den Aufnahmekörper bereits vollständig durchdrungen hat.

Entsprechend einer anderen günstigen Weiterbildung der Erfindung sind die Teilelemente des Aufnahmekörpers als Längsschnitt-Hälften eines Hohlzylinders mit jeweils einer Ausnehmung ausgebildet, welche bei zusammengesetztem Aufnahmekörper einen im wesentlichen rechteckigen Hohlraum begrenzen. An der Seite, an welcher der Spreizkörper eingebracht wird, tragen die Teilelemente jeweils einen flanschförmigen Kragen, durch welchen eine genaue und sichere Plazierung des Aufnahmekörpers in der in den zu verbindenden Knochenteilen vorgesehenen Bohrung ermöglicht wird. Der Kragen verhindert auf einfache Weise gleichermaßen, daß der Aufnahmekörper in die Bohrung gedrückt wird, wenn das Eintreiben des Spreizkörpers erfolgt.

Die formschlüssige Verankerung der Teilelemente des Aufnahmekörpers in dem Knochenmaterial wird in günstiger Weise durch eine profilierte Oberfläche von deren äußerer Wandung erreicht. Hierfür sind halbringförmig angeordnete Nuten vorgesehen, welche einen dreieckigen Querschnitt aufweisen. Die Anordnung der Nuten ist derart gewählt, daß ein Längsschnitt durch die Teilelemente eine sägezahnförmige Begrenzungslinie aufweist.

Gemäß einer anderen Weiterbildung der Erfindung weisen die Teilelemente ein unterschiedliches Querschnittsprofil auf. Dabei ist bei einem Teilelement an der Peripherie eine sich über die gesamte Länge erstreckende Abplattung vorgesehen. Dadurch ergibt sich in vorteilhafter Weise bei einem in die Knochenbohrung eingesetzten Aufnahmekörper zwischen den jeweiligen Wandungen ein zusätzlicher Freiraum, in welchem ein weiteres an dem Knochen dauerhaft zu befestigendes Element plazierbar ist. Die Lösung schafft beispielsweise günstige osteosynthetische Bedingungen für eine Kniegelenk-Operation, bei der zur Kreuzbandbefestigung ein Knochenstück mit angewachsener Sehne in einen derartigen Freiraum eingesetzt und durch Aufspreizen des Aufnahmekörpers verankert werden kann.

Sowohl der Aufnahmekörper als auch der Spreizkörper bestehen aus einem Werkstoff, welcher bei akzeptabler Biokompatibilität einem resorbierenden Abbau unterliegt und werden durch Spritzgießen gefertigt. Als Werkstoff ist vorzugsweise ein Polylactid, ein Polyglykolid oder ein rechts- bzw. linksdrehendes Copolymer dieser Stoffe vorgesehen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine Seitenansicht der bevorzugten Ausführungsform der Erfindung in nicht gespreiztem Zustand,
Figur 1a die Ansicht von rechts entsprechend der Darstellung in Figur 1,
Figur 2 eine Seitenansicht der bevorzugten Ausführungsform der Erfindung nach Eintreiben eines (nicht dargestellten) Spreizkörpers`
Figur 2a die Ansicht von rechts entsprechend der Darstellung in Figur 2,
Figur 3 eine günstige Ausführungsform eines Spreizkörpers in Ansicht von der Seite sowie
Figur 4 eine Draufsicht auf einen Knochenbereich mit eingesetztem Befestigungselement in schematisierter Darstellung.

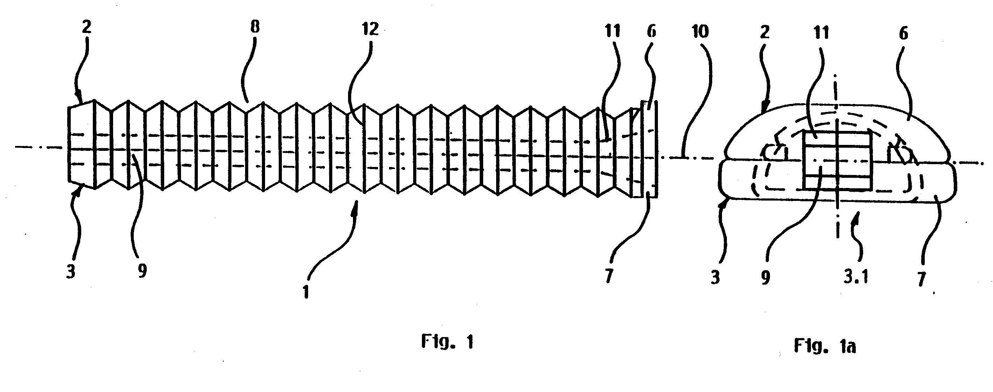

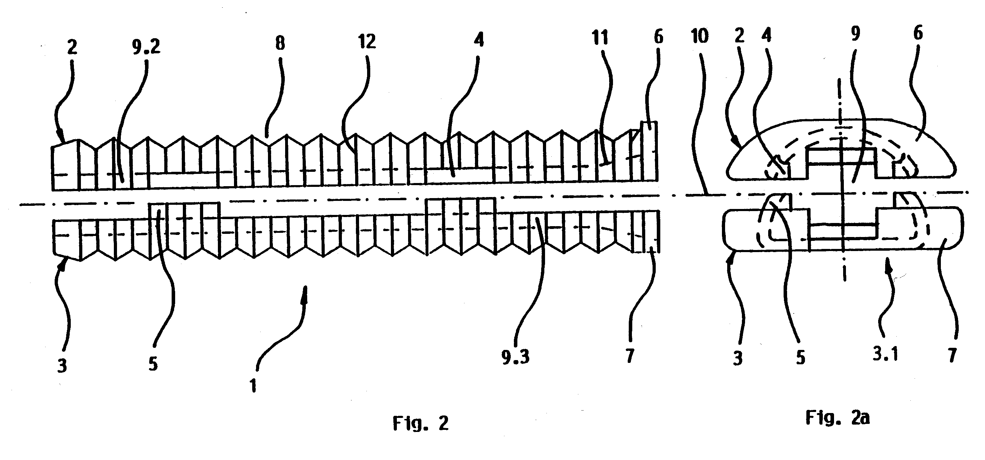

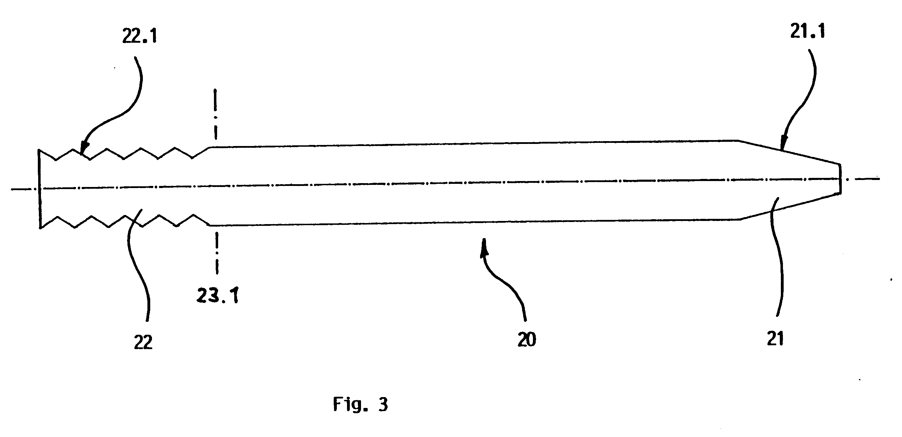

Der in den Figuren 1 und 1a dargestellte Aufnahmekörper 1 ist aus zwei unterschiedlich ausgebildeten, stabförmigen Teilelementen 2 und 3 mit einer gleichgroßen, sich axial erstreckenden und rechteckigen Ausnehmung (vergleiche die Positionen 9.2 und 9.3 in den Figuren 2 und 2a) zusammengesetzt. Die Teilelemente 2, 3 sind miteinander nicht fest verbunden. Sie werden durch Führungselemente (vergleiche die Positionen 4 und 5 in den Figuren 2 und 2a) in der dargestellten Position gehalten und bilden einen hohlzylindrischen Körper, welcher einseitig abgeplattet ist. Die Ausnehmungen der Teilelemente 2, 3 begrenzen einen Hohlraum 9 mit rechteckförmigen Querschnitt, welcher sich über die gesamte Länge des Aufnahmekörpers 1 erstreckt. An einem Endbereich des Hohlraums 9 ist eine keilförmige Erweiterung 11 in Richtung der Hohlraumöffnung vorgesehen. Dadurch wird das Eintreiben eines als länglicher Quader ausgebildeten Spreizkörpers (nicht dargestellt, vergleiche Position 20 in Figur 3) während des chirurgischen Eingriffs erheblich erleichtert.

Durch die einseitige Abplattung 3.1 an der Peripherie des Teilelements 3 steht in vorteilhafter Weise ein Freiraum zwischen dem in eine Knochenbohrung eingesetzten Aufnahmekörper 1 und der Wandung dieser Bohrungbereit, in welchen ein zusätzliches osteosynthetisch zu verbindendes Element vor Eintreiben des Spreizkörpers einbringbar ist. Diese Form des Befestigungselementes schafft somit günstige Bedingungen für Kniegelenkoperationen, wo eine Kreuzbandbefestigung vorgesehen ist.

Jedes der Teilelemente 2, 3 trägt jeweils an dem gleichen Ende einen flanschförmigen Kragen 6, 7. Dieser Kragen sichert, daß ein im Zusammenhang mit einem osteosynthetischen Eingriff in eine Knochenbohrung eingebrachter Aufnahmekörper 1 in der gewünschten Position verbleibt, wenn der Spreizkörper in den Hohlraum 9 längs der Achse 10 eingetrieben wird.

Die an der Peripherie der Teilelemente 2, 3 vorgesehene Profilierung dient bei einem in eine Knochenbohrung eingesetzten Aufnahmekörper 1 zu dessen Verankerung und zur günstigen Krafteinleitung in den Knochen, wenn der Spreizkörper in den Hohlraum 9 eingetrieben wird. Sie wird durch gleichartige, dreieckeckige Nuten 8 gebildet, welche die Teilelemente jeweils halbringförmig umgreifen und dabei in umlaufenden Kanten 12 zusammengeführt sind.

Der in den Figuren 2 und 2a gezeigte Aufnahmekörper 1 ist durch einen in den Hohlraum 9 eingetriebenen (nicht dargestellten) Spreizkörper in seiner Raumform vergrößert. Die Teilelemente 2, 3 sind dabei in entgegengesetzter Richtung und jeweils um den gleichen Betrag senkrecht zur Hohlraumachse 10 verschoben worden. Diese Bewegung wird durch die eine Zunge 5 und eine rechteckige Ausnehmung 4 aufweisende Gleit-Führung sowie durch die rechteckige Ausbildung der den Kanal 9 begrenzenden Ausnehmungen 9.2, 9.3 bzw. des stabförmigen Spreizkörpers erzwungen und sichert ein gleichmäßiges Anpressen der profilierten Wandung der Teilelemente 2, 3 an das Knochenmaterial über die gesamte Länge des Befestigungselements.

Der in Figur 3 dargestellte Spreizkörper 20 des Befestigungselements besteht ebenso wie die Teilelemente des Aufnahmekörpers aus einem Material, welches einem resorbierenden Abbau im menschlichen Körper unterliegt. Der stabförmige Spreizkörper weist ein rechteckiges Querschnittsprofil auf und geht an einem seiner Enden 21 in eine keilförmige ausgebildete Anfasung 21.1 über, die das Eintreiben des Spreizkörpers in einen im Knochenmaterial positionierten Aufnahmekörper wesentlich erleichtert. An dem anderen Ende 22 ist eine Profilierung 22.1 vorgesehen, um ein Werkzeug zur Handhabung des Spreizkörpers bei einem osteosynthetischen Eingriff sicher ansetzen zu können. Die Profilierung besteht aus Rippen mit dreieckigem Querschnitt, welche sich quer zur Längsachse des Spreizkörpers erstrecken.

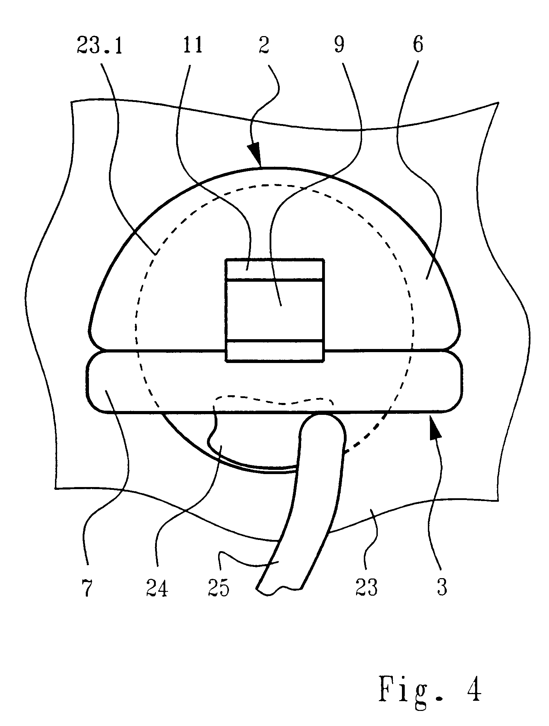

Der in Figur 4 dargestellte Knochenbereich 23 weist eine Bohrung 23.1 auf, in welche der aus den Teilelementen 2 und 3 bestehende Aufnahmekörper eingesetzt ist. Durch die Abplattung an der Peripherie des Teilelements 3 verbleibt dabei zwischen der Bohrungswandung und dem Aufnahmekörper ein Freiraum. Dieser ist zum Einsetzen von bei einem osteosynthetischen Eingriff zu befestigenden Elementen 24, 25 nutzbar. So bietet der Freiraum beispielsweise bei einer Kniegelenk-Operation mit Kreuzband-Ersatz günstige Bedingungen zum Plazieren eines Knochenstücks 24 mit einer daran befestigten Sehne 25. Nach Eintreiben des (nicht dargestellten) Spreizkörpers werden Knochenstück 24 und Sehne 25 durch das Teilelement 3 verankernd gegen die Knochenwandung gepreßt.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei anders gearteten Ausführungen Gebrauch macht.

## Patentansprüche

1. Osteosynthetisches Befestigungselement, bestehend aus einem in eine Bohrung einbringbaren Aufnahmekörper (1) und einem Spreizkörper (20), wobei der Aufnahmekörper durch Eintreiben des Spreizkörpers in eine in dem Aufnahmekörper vorgesehene längsgerichtete Bohrung oder andere eine umlaufende Wandung aufweisende Öffnung (9; 9.2, 9.3) durch Keilwirkung radial expandierbar ist,
**dadurch gekennzeichnet,**
daß der in Längsrichtung geteilte Aufnahmekörper (1) aus mindestens zwei getrennten oder derart beweglich miteinander verbundenen Teilelementen (2, 3) besteht, daß der Aufnahmekörper durch Eintreiben des Spreizkörpers infolge der gegenseitigen Keilwirkung von Aufnahmekörper und Spreizkörper im wesentlichen über seine ganze Länge gleichmäßig expandierbar ist.

2. Befestigungselement nach Anspruch 1, **dadurch gekennzeichnet**, daß die Teilelemente (2, 3) sich zu einem Körper (1) mit im wesentlichen zylindrischer Außenwandung mit gleichbleibendem Querschnitt ergänzen.

3. Befestigungsmittel nach Anspruch 2, **dadurch gekennzeichnet**, daß der innere Hohlraum (9; 9.2, 9.3) des Zylinders einen im wesentlichen polygonalen Querschnittt, aufweist.

4. Befestigungselement nach Anspruch 2 oder 3, **dadurch gekennzeichnet**, daß die Teilelemente (2, 3) im äußeren Peripheriebereich mindestens über einen Teil ihrer Längserstreckung eine Profilierung (8, 12) oder Strukturierung aufweisen, welche sich im wesentlichen quer zur Eintreibrichtung des Befestigungselements in den Knochen erstreckt und insbesondere aus die Teilelemente im wesentlichen halbringförmig umgreifenden Nuten (8) mit dreieckigem Querschnitt besteht.

5. Befestigungselement nach Anspruch 1, **dadurch gekennzeichnet**, daß mindestens zwei der Teilelemente (2, 3) unterschiedliche Querschnittsformen aufweisen.

6. Befestigungselement nach Anspruch 5, **dadurch gekennzeichnet**, daß mindestens ein Teilelement (3) eine Abplattung (3.1) seiner äußeren Verrundung aufweist.

7. Befestigungselement nach Anspruch 1, **dadurch gekennzeichnet**, daß mindestens eines der Teilelemente (2, 3) mit einem flanschartigen Kragen (6, 7) versehen ist.

8. Befestigungselement nach Anspruch 1, **dadurch gekennzeichnet**, daß eine sich tangierend erstreckende Zunge (5) an einem (3) der Teilelemente vorgesehen ist, welche zum Eingriff in eine an dem anderen Teilelement (2) zur führenden Aufnahme der Zunge vorgesehene Ausnehmung (4) ausgestattet ist.

9. Befestigungselement nach Anspruch 7, **dadurch gekennzeichnet**, daß sich die Öffnung (9; 9.2, 9.3) zum den Kragen (6, 7) tragenden Ende der Teilelemente (2, 3) geringfügig keilförmig erweitert.

10. Befestigungselement nach Anspruch 1, **dadurch gekennzeichnet**, daß das einen geringeren Querschnitt aufweisende Ende (21) des Spreizkörpers (20) eine im wesentlichen keilförmige Anfasung (21.1) aufweist und/oder die Länge des Spreizkörpers die Länge der Teilelemente (2, 3) überschreitet.

11. Befestigungselement nach Anspruch 10, **dadurch gekennzeichnet**, daß das beim Eintreiben rückwärtige Ende (22) des Spreizkörpers (20) eine Profilierung zur Verbesserung des Angriffs eines Werkzeugs aufweist.

12. Befestigungselement nach Anspruch 11, **dadurch gekennzeichnet**, daß die Profilierung an zwei einander gegenüberliegenden Seitenflächen vorgesehen ist und sich quer zur Längsachse des Spreizkörpers (20) erstreckende Profilelemente (22.1) mit einem vorzugsweise dreieckigen Querschnitt aufweist.

13. Befestigungselement nach Anspruch 1, **dadurch gekennzeichnet**, daß es aus einem der resorbierbaren Werkstoffe Polylactid, Polyglykolid oder ein rechts- bzw. linksdrehendes Copolymer dieser Stoffe gefertigt ist.

## Claims

1. Osteosynthetic securing element comprising a receiving member (1), which may be inserted into a hole, and a spreading member (20), whereby the receiving member may be expanded radially by wedge effect by driving the spreading member into a longitudinally directed hole provided in the receiving member or into another opening (9; 9.2, 9.3) having a circumferential wall, characterised in that the receiving member (1) divided in the longitudinal direction comprises at least two separate part-elements (2, 3) or part-elements which are movably connected to one another in such a way that as a result of the mutual wedge effect of the receiving member and spreading member, the receiving member may be uniformly expanded essentially over its entire length by driving in the spreading member.

2. Securing element according to Claim 1, characterised in that the part-elements (2, 3) supplement one another to form a member (1) with an essentially cylindrical external wall with constant cross-section.

3. Securing means according to Claim 2, characterised in that the internal cavity (9; 9.2, 9.3) of the cylinder has an essentially polygonal cross-section.

4. Securing element according to Claim 2 or 3, characterised in that at least over a portion of their longitudinal extent in the outer peripheral region, the part-elements (2, 3) have profiling (8, 12) or structuring, which extends essentially transversely to the direction in which the securing element is driven into the bone, and in particular comprises grooves (8) engaging around the part-elements essentially in a half ring shape and having a triangular cross-section.

5. Securing element according to Claim 1, characterised in that at least two of the part-elements (2, 3) have different cross-sectional shapes.

6. Securing element according to Claim 5, characterised in that at least one part-element (3) has a flattened portion (3.1) on its external curvature.

7. Securing element according to Claim 1, characterised in that at least one of the part-elements (2, 3) is provided with a flange-like collar (6, 7).

8. Securing element according to Claim 1, characterised in that a tongue (5) extending at a tangent is provided on one (3) of the part-elements, said tongue being equipped for engagement into a recess (4) provided on the other part-element (2) for guiding and receiving the tongue.

9. Securing element according to Claim 7, characterised in that the opening (9; 9.2, 9.3) widens slightly in a wedge shape to the end of the part-elements (2, 3) supporting the collar (6, 7).

10. Securing element according to Claim 1, characterised in that the end (21) of the spreading member (20) having a smaller cross-section has an essentially wedge-shaped chamfer (21.1) and/or the length of the spreading member exceeds the length of the part- elements (2, 3).

11. Securing element according to Claim 10, characterised in that the end (22) of the spreading member (20) at the rear while being driven in has profiling for improving the working engagement of a tool.

12. Securing element according to Claim 11, characterised in that the profiling is provided on two opposing side faces and has profile elements (22.1) extending transversely to the longitudinal axis of the spreading member (20) and having a preferably triangular cross-section.

13. Securing element according to Claim 1, characterised in that it is made from one of the resorbable substances polylactide, polyglycolide or a dextro- or laevo-rotatory copolymer of these substances.

## Revendications

1. Elément de fixation ostéosynthétique, composé d'un corps de réception (1) pouvant être introduit dans un trou foré et d'un corps d'écartement (20), le corps de réception pouvant être expansé radialement par enfoncement par effet de coin du corps d'écartement dans un trou foré longitudinal ou autre ouverture présentant une paroi circonférentielle (9; 9.2, 9.3) prévus dans le corps de réception,
caractérisé en ce que le corps de réception (1) divisé dans le sens longitudinal se compose d'au moins deux éléments partiels (2, 3) séparés ou reliés entre eux de façon mobile, de telle sorte que le corps de réception peut être expansé régulièrement sensiblement sur toute sa longueur par enfoncement du corps d'écartement par suite de l'effet de coin mutuel du corps de réception et du corps d'écartement.

2. Elément de fixation selon la revendication 1, caractérisé en ce que les éléments partiels (2, 3) se complètent pour former un corps (1) ayant une paroi externe sensiblement cylindrique de section transversale constante.

3. Moyen de fixation selon la revendication 2, caractérisé en ce que la cavité interne (9; 9.2, 9.3) du cylindre présente une section transversale sensiblement polygonale.

4. Elément de fixation selon la revendication 2 ou 3, caractérisé en ce que les éléments partiels (2, 3) présentent dans leur zone périphérique externe, au moins sur une partie de leur étendue longitudinale, un profilage (8, 12) ou structuration qui s'étend sensiblement perpendiculairement au sens d'enfoncement de l'élément de fixation dans l'os et se compose en particulier de gorges (8) à section triangulaire entourant sous forme sensiblement semi-annulaire les éléments partiels.

5. Elément de fixation selon la revendication 1, caractérisé en ce qu'au moins deux des éléments partiels (2, 3) présentent des sections transversales de formes différentes.

6. Elément de fixation selon la revendication 5, caractérisé en ce qu'au moins un élément partiel (3) présente un aplatissement (3.1) de sa rotondité externe.

7. Elément de fixation selon la revendication 1, caractérisé en ce qu'au moins un des éléments partiels (2, 3) est doté d'un collet (6, 7) en forme de bride.

8. Elément de fixation selon la revendication 1, caractérisé en ce qU'une languette s'étendant tangentiellement (5) est prévue sur un (3) des éléments partiels qui est équipé pour l'emboîtement dans un évidement (4) prévu sur l'autre élément partiel (2) pour la réception à réaliser de la languette.

9. Elément de fixation selon la revendication 7, caractérisé en ce que l'ouverture (9; 9.2, 9.3) est élargie légèrement en forme de coin à l'extrémité des éléments partiels (2, 3) portant le collet (6, 7).

10. Elément de fixation selon la revendication 1, caractérisé en ce que l'extrémité (21) du corps d'écartement (20) présentant une section transversale plus faible présente un biseau (21.1) sensiblement cunéiforme et/ou la longueur du corps d'écartement dépasse la longueur des éléments partiels (2, 3).

11. Elément de fixation selon la revendication 10, caractérisé en ce que l'extrémité (22) du corps d'écartement (20) se trouvant à l'arrière lors de l'enfoncement présente un profilage pour l'amélioration de l'attaque d'un outil.

12. Elément de fixation selon la revendication 11, caractérisé en ce que le profilage est prévu sur deux faces latérales opposées et présente des éléments profilés (22.1) de section de préférence triangulaire s'étendant perpendiculairement à l'axe longitudinal du corps d'écartement (20).

13. Elément de fixation selon la revendication 1, caractérisé en ce qu'il est fabriqué en l'une des matières résorbables polylactide, polyglycolide ou un copolymère lévogyre ou dextrogyre de ces substances.
